# FASCICULE DE BREVET EUROPEEN

(11) **EP 2 030 017 B1**
(45) Date de publication et mention de la délivrance du brevet: **18.04.2012**
(21) Numéro de dépôt: 07788927.7
(22) Date de dépôt: 25.06.2007
(51) Int. Cl.: G01N 33/53, G01N 33/543, C12Q 1/56, G01N 33/86

(54) **PROCÉDÉ ET NÉCESSAIRE POUR LA DÉTECTION DES ANTICORPS HÉPARINE-DÉPENDANTS ET LE DIAGNOSTIC DES PATHOLOGIES IMMUNES OU AUTO-IMMUNES POTENTIALISÉES PAR L'HÉPARINE TELLE QUE LA THROMBOPÉNIE INDUITE PAR L'HÉPARINE**
VERFAHREN UND KIT FÜR DEN NACHWEIS HEPARINABHÄNGIGER ANTIKÖRPER UND DIE DIAGNOSE VON HEPARIN-POTENZIERTEN IMMUN- BZW. AUTOIMMUNERKRANKUNGEN WIE ETWA HEPARIN-INDUZIERTE THROMBOPENIE
METHOD AND KIT FOR THE DETECTION OF HEPARIN-DEPENDENT ANTIBODIES AND THE DIAGNOSIS OF IMMUNE OR AUTOIMMUNE PATHOLOGIES POTENTIATED BY HEPARIN SUCH AS HEPARIN-INDUCED THROMBOCYTOPENIA

(30) Priorité: 23.06.2006 FR 0605662
(43) Date de publication de la demande: 04.03.2009
(73) Titulaire: Hyphen Biomed, 95000 Neuville sur Oise (FR)
(72) Inventeur: AMIRAL, Jean, 95000 Neuville sur Oise (FR); VISSAC, Anne-Marie, 95000 Neuville sur Oise (FR)
(74) Mandataire: Thomas, Nadine
(86) Numéro de dépôt international: PCT/FR2007/001049
(87) Numéro de publication internationale: WO 2007/147983

(56) Documents cités:
- US-A- 5 753 445
- US-A- 5 972 717
- US-A- 5 972 717
- FRANCIS J L: "A CRITICAL EVALUATION OF ASSAYS FOR DETECTING ANTIBODIES TO THE HEPARIN-PF4 COMPLEX" SEMINARS IN THROMBOSIS AND HEMOSTASIS, STUTTGART, DE, vol. 30, no. 3, 2004, pages 359-368, XP008062028 ISSN: 0094-6176
- AMIRAL J ET AL: "ANTIBODIES TO MACROMOLECULAR PLATELET FACTOR 4-HEPARIN COMPLEXES IN HEPARIN-INDUCED THROMBOCYTOPENIA: A STUDY OF 44 CASES", THROMBOSIS AND HAEMOSTASIS, SCHATTAUER GMBH, DE; US, vol. 73, no. 1, 1 January 1995 (1995-01-01), pages 21-28, XP008077533, ISSN: 0340-6245

## Description

La présente invention concerne un procédé rapide, efficace, économique, et sensible de détection d'anticorps induits par une substance héparinique et de diagnostic de pathologies immunes ou auto-immunes potentialisées par une substance héparinique telle que la thrombopénie induite par l'héparine (TIH de type II). L'invention concerne également un nécessaire de détection des anticorps héparine-dépendants et de diagnostic de pathologies potentialisées par une substance héparinique telle que la thrombopénie induite par l'héparine.

Les thrombopénies ou thrombocytopénies peuvent être de plusieurs origines. Elles peuvent être provoquées par la présence de médicaments notamment la quinine/quinidine, le polysulfate de pentosane mais surtout l'héparine. *(voir l'article de* M.C.BERNDT et al, Blood Reviews 1 pages 111-118, (1987*) et l'article de* B.TARDY-PONCET, Am JHematol 2994 ; 45(3) : 252-7*)*

L'héparine est administrée aux patients en tant que facteur anti-coagulant pour prévenir les risques de thromboses veineuses ou artérielles. Cependant, certains patients traités à l'héparine développent des anticorps induits par l'héparine entraînant une thrombopénie qui peut être très sévère voire fatale. Ces thrombopénies semblent provoquées par des anticorps IgG, IgM ou IgA qui se développent après 5 jours ou plus de traitement à l'héparine. Les anticorps d'isotype IgG sont de plus loin les plus pathogènes.

Cette réaction survient à une « concentration critique » d'héparine *(*Gruel Y, Presse Med 1998 ; 27 (suppl 2) : 7-12 *et* Warkentin TE et al, Chest 2004 ; 126 : 311S-37S*.)*

Il apparaît essentiel d'identifier rapidement les patients à risque ou développant cette pathologie de TIH de type II.

Actuellement, les méthodes de diagnostic de thrombopénie sont :
- la numération des plaquettes sanguines avant, pendant et après le traitement, méthode longue et peu spécifique.
- la recherche d'une absence d'étiologie différente des thrombopénies (infection, autres thérapies...) , méthode longue et fastidieuse.
- l'utilisation de tests biologiques recherchant la présence d'anticorps dirigés contre les plaquettes en présence du médicament inducteur.
- la recherche et la mesure par méthode ELISA ou toute autre méthode immunologique des anticorps dirigés contre les complexes d'héparine et de facteur plaquettaire 4 (pF4).

Parmi ces tests biologiques, on utilise surtout les tests d'agrégation plaquettaire qui nécessitent un appareillage adapté et dont les modalités opératoires sont longues et manquent de sensibilité.

Les autres méthodes qui ont été décrites *[articles de* J. G. KELTON et al, Blood 72 (No2), pages 925-930, (1988*) et* B.H. CHONG, British Journal of Haematology 49, pages 531-540, (1988*) et* B.H. CHONG, Blood Reviews 2, pages 108-114, (1986*) et* D.SHERIDAN et al, Blood 67 (No1), pages 27-30, (1986*) et* Y.GRUEL, Sang Thrombose Vaisseaux, 1 (No4), pages 233-236, (1989*)]* mettent en oeuvre l'étude de la fixation plaquettaire des Ig G sériques, la libération de la sérotonine ¹⁴C-radiomarquée à deux concentrations différentes d'héparine, la disponibilité du facteur plaquettaire 4, la fixation du complément, l'inhibition de la lyse du complément et l'agglutination d'hématies sensibilisées. Ces méthodes présentent les inconvénients d'être peu sensibles ou longues à réaliser.

D'autres méthodes sensibles et rapides dans leur réalisation, basées sur la cytochimie, utilisent soit des marquages des plaquettes activées ou non, détectées par cytométrie de flux, soit des facteurs purifiés issus des plaquettes pouvant se lier préférentiellement à l'héparine et formant un complexe avec les anticorps induits par l'héparine, soit des tests de compétitions de liaison. Tous ces tests sont réalisés avec des plaquettes fraîches. Enfin d'autres tests immunocytochimiques sont effectués avec de l'héparine, du plasma à tester contenant potentiellement les anticorps induits par l'héparine et un anticorps couplé à une molécule rapporteur mais ces derniers tests sont réalisés sans ajout de plaquettes pourtant nécessaires à la sensibilisation du test.

Dans la prévention ou le traitement de la TIH de type II, le dosage des anticorps induits par l'héparine est indispensable.

Il existe plusieurs types d'héparines utilisés :
- les héparines non fractionnées (HNF) qui induisent plus tôt et plus fréquemment l'apparition d'anticorps induits par l'héparine
- les héparines de bas poids moléculaires.

Il est toutefois possible d'utiliser tout type de dérivés hépariniques ou des polymères linéaires non-glycosaminoglycan chargés négativement n'étant pas un hydrate de carbone comme le polyvinylsulfate, le polyvinylsulfonate, le polystyrène sulfonate, le polyanétholsulfonate, des polyvinyl phosphate et polyvinylphosphonate, le poly-D glutamate *(voir PCT*/*US97*/*02840 WO9732211).*

Il a été découvert que l'utilisation de facteurs ayant une grande affinité pour l'héparine sont très intéressants car ils permettent aux tests biologiques d'être plus spécifiques et sensibles. En effet, très longtemps, les anticorps induits par l'héparine ont été considérés comme étant dirigés contre l'héparine elle-même mais depuis que la cible antigénique des anticorps a été identifiée, notamment tel que décrit dans EP 0 495 971, comme étant lest complexes stoechiométriques d'héparine et de facteur plaquettaire, pF4, de plus en plus d'informations sur les mécanismes impliqués sont disponibles. En effet, le pF4 en se liant à l'héparine, pour neutraliser ses propriétés anticoagulantes, change de conformation et devient alors immunogène.

Dans ce procédé, les facteurs issus des plaquettes ayant une forte affinité pour l'héparine ou la drogue inductrice d'anticorps et obtenus par clivage ou lyse plaquettaire sont :
- le facteur plaquettaire 4 ou pF4
- des fractions de ce facteur pF4
- des fractions contenant au moins une substance éluée en même temps que le pF4
- le pF4 recombinant et ses variants
- des peptides synthétiques reprenant toute ou partie de la séquence des aminoacides du pF4
- le protéoglycane
- des complexes protéoglycane-pF4
- ainsi que leurs mélanges.

Toutefois, un tel procédé nécessite obligatoirement la purification du facteur plaquettaire 4 (pF4), ou l'utilisation de pF4 recombinant purifié et fonctionnel, ce qui rend plus complexe et très onéreux le procédé.

Le brevet US 5972717 décrit un tel procédé de détection des anticorps induits par l'héparine utilisant un facteur à forte affinité pour l'héparine adsorbé sur un support, du facteur plaquettaire pF4 purifié et le sérum ou le plasma d'un patient à tester. Dans ce procédé, le facteur à forte affinité pour l'héparine utilisé est la streptavidine et elle est utilisée dans le but de fixer la molécule d'héparine bien sûr mais surtout de l'orienter sur le support. Par ailleurs, la mise en oeuvre du procédé tel que décrit nécessitait une purification obligatoire du pF4, un inconvénient majeur en terme de complexité, de fiabilité mais aussi de coût.

Un autre procédé dans la détection des anticorps induits par l'héparine est décrit dans le document intitulé « Antibodies to macromolecular platelet factor 4-heparin complexes in heparin-induced thrombocytopenia : a study of 44 cases » publié dans Thrombosis and haemostasis, vol.73, no.1, 1995, pages 21 à 28. Ce document décrit pour détecter les anticorps se liant au complexe pF4-héparine est un test ELISA utilisant une substance (MBSS) revêtant les puits de la plaque, un mélange de pF4-héparine, un sérum de chèvre et le plasma à tester. Bien qu'un tel procédé soit intéressant, il présente cependant quelques limites, principalement quant à son coût et sa détection. En effet, ce test ELISA nécessite une purification indispensable de l'antigène pF4, un inconvénient déjà exposé précédemment. De plus, ce procédé tel que décrit ne permet qu'une détection des anticorps dépendants du pF4 or, dans le diagnostic de pathologies immunes ou auto-immunes induites par l'héparine, il serait particulièrement intéressant et avantageux de pouvoir détecter tous les anticorps induits par l'héparine c'est-à-dire tous les anticorps induits par les complexes antigène-héparine, l'antigène ne se limitant pas qu'au pF4.

La majorité des procédés récents de l'art antérieur concernant cette détection d'anticorps induits par l'héparine utilisent la fixation de ladite héparine sur un support et l'ajout d'une substance antigénique, telle que par exemple le facteur plaquettaire pF4, des polymères glycosaminoglycans ou protéoglycanes, permettant la formation du complexe générant des anticorps héparine dépendants détectables. Néanmoins, tous les documents de l'art antérieur (US 5972717, US 5753445, US 5972718, US 5466582, la publication intitulée « Antibodies to macromolecular platelet factor 4-heparin complexes in heparin-induced thrombocytopenia : a study of 44 cases » et publiée dans Thrombosis and haemostasis, vol.73, no.1, 1995, pages 21 à 28, la publication intitulée « Polyarginine as a multifunctional fusion tag » publiée dans Protein science : a publication of the protein society jun 2005, vol. 14, no. 6, juin 2005 (2005-06), pages 1538-1544) nécessitent au moins une étape de purification du matériel utilisé ce qui augmente considérablement le coût du procédé. Aussi, à la lumière de l'art antérieur exposé ci-dessus, il était nécessaire de mettre au point un procédé de détection des anticorps induits par l'héparine à coût réduit et à sensibilité accrue.

La présente invention a donc pour objet de proposer un procédé de détection et de diagnostic permettant d'obtenir, dans ce genre de tests en immunocytochimie, une meilleure sensibilité et de très bons résultats de détection d'anticorps et de diagnostic de pathologies immunes ou auto-immunes potentialisées par une substance héparinique dont la TIH de type II , tout en étant économiquement plus avantageux.

Ainsi, pour ce faire, est proposé dans un premier mode de réalisation, un procédé selon la revendication 1.

Selon un deuxième mode de réalisation, est également proposé un procédé selon la revendication 2.

Par simplicité de lecture, la dénomination anti(Ag-SH) englobera l'anticorps anti(Ag-SH) ainsi que la forme complexe immun complexe Ag-SH-anti(Ag-SH) présents potentiellement dans le sérum ou le plasma à tester. Ce complexe immun est capable de réagir avec le complexe SFA-SH décrit précédemment. Ce procédé de détection selon l'invention permet donc également de doser des complexes immuns circulants dans le sérum ou le plasma du patient.

Selon le premier mode de réalisation de l'invention, au moins une substance à forte affinité pour une substance héparinique (SFA), telle que, de préférence, le sulfate de protamine, la streptavidine (dans ce cas la substance héparinique est obligatoirement biotinylée, la streptavidine étant affine à l'héparine par le biais de la biotine), la poly-L-lysine, la poly-arginine, le polybrène, immobilise au moins une substance héparinique en excès (SH) qui, à son tour va fixer les molécules affines pour la substance héparinique (dont le pF4) en formant ainsi le complexe (Ag-SH). Ce complexe (Ag-SH) sera reconnu par les anticorps spécifiques dirigés contre lui anti(Ag-SH) et contenus dans le plasma ou le sérum du patient à tester formant le complexe (Ag-SH)-anti(Ag-SH).

Ce même complexe sera détecté par des techniques diverses et connues de l'homme du métier, détaillées plus loin. En comparaison avec les approches précédentes, cette approche avec la substance à forte affinité pour une substance héparinique comme le sulfate de protamine, la streptavidine, la poly-L-lysine, la poly-arginine, le polybrène, présente les principaux avantages de la rapidité, de la spécificité et la détection de tous les anticorps héparine-dépendants (et pas seulement dépendants du pF4), d'un coût de mise en oeuvre très compétitif, d'une grande sensibilité et d'une possibilité de déclinaison dans des techniques individuelles ultra-rapides.

Cette méthode de détection peut être déclinée en plusieurs technologies connues : test ELISA, immuno-turbidimétrie, agglutination de latex sur lame, néphélométrie, turbidimétrie ou néphélométrie amplifiée par des particules de latex, immunofluorescence (lame, microplaque, latex fluorescent, latex magnétique, test sur membranes, biochips, etc...), et de façon générale tout procédé permettant d'identifier et/ou de quantifier une réaction entre un antigène (y compris les haptènes) et un anticorps, de quelque isotype qu'il soit.

La ou les substances antigéniques potentielles capables de réagir avec la substance héparinique pour former le complexe (Ag-SH) peuvent provenir directement du plasma ou du sérum du patient à tester. Toutefois, dans ce cas, la concentration de ces substances peut alors être un facteur limitant la sensibilité du test.

En conséquence, selon une première variante préférée du procédé selon l'invention, la au moins une substance antigénique potentielle peut être amenée en excès par un lysat plaquettaire consistant en un concentré de plaquettes normales (mélange de plusieurs donneurs par exemple), lysées (par destruction des plaquettes) ou un lysat leucocytaire (concentré de leucocytes normaux). Cette variante permet une sensibilisation maximale du procédé, la ou les substances antigéniques potentielles étant amenées en quantité suffisante et, de préférence en excès, pour pouvoir réagir avec la substance héparinique pour former le complexe (Ag-SH).

Le cas échéant, si le concentré plaquettaire ou le concentré leucocytaire ou l'échantillon testé du patient ne contenaient pas en quantité suffisante la substance affine pour la substance héparinique, et antigène potentiel, celle-ci pourrait être amenée par tout milieu biologique la contenant. Ainsi, cette technique est extensible à tout antigène formé par complexation avec la substance héparinique, ou toute substance ayant des propriétés analogues, telles que définies précédemment, et capable de réagir avec les anticorps présents dans les états pathologiques immuns ou auto-immuns potentialisés par une substance héparinique, comme la thrombopénie induite par l'héparine.

Le procédé de détection de la présente invention se déroule selon les étapes suivantes :
1) Fixation par adsorption ou par liaison covalente d'au moins une substance ayant une forte affinité pour une substance héparinique (SFA) sur un support essentiellement solide, ou tout autre support réactionnel permettant de réaliser un test immunologique. Cette SFA est le plus souvent du sulfate de protamine car il présente l'avantage d'être très bon marché et facilement disponible ; en variante, la fixation peut concerner la streptavidine (la substance héparinique étant alors biotinylée) mais également la poly-L-lysine ou la poly-arginine, selon les mêmes procédés que pour le sulfate de protamine.
2) Ajout d'au moins une substance héparinique (le cas échéant biotinylée) en excès.
3) Formation d'un complexe substance à forte affinité pour la substance héparinique-substance héparinique (soit SFA-SH). Au moins un lavage pour retirer l'excès d'héparine.
4) De façon optionnelle, sensibilisation si nécessaire par ajout d'un lysat plaquettaire global provenant d'un concentré plaquettaire préparé à partir des plaquettes de donneurs normaux ou un lysat leucocytaire, et contenant des molécules affines pour la substance héparinique potentiellement antigéniques et appelées substances antigéniques.
5) Formation d'un complexe substance antigénique-substance héparinique (soit Ag-SH).
6) Ajout du plasma ou du sérum du patient à tester contenant :
   a) des substances se complexant à la substance héparinique pour devenir potentiellement antigéniques et
   b) potentiellement des anticorps induits par l'héparine anti(Ag-SH), éventuellement sous forme également de complexes immuns.
7) Suivant le sérum ou le plasma testé, formation des complexes (Ag-SH)-anti(Ag-SH). Au moins un lavage pour éliminer toute substance pouvant interférer avec le système de détection.
8) Ajout des anticorps dirigés contre les anticorps anti-(Ag-SH). Ces anticorps sont détectables par marquage selon des techniques connues décrites plus bas (enzymo-immunologie, radio-immunologie ...).
9) Formation des complexes (Ag-SH)-anti(Ag-SH)-anti(anti-Ag-SH). Lavages
10) Révélation des marquages par les techniques immunologiques ou toute autre méthode permettant d'identifier la formation de complexes. Détection et dosage des anticorps induits par la substance héparinique et responsables des thrombopénies induites par l'héparine (TIH de type II).
11) Diagnostic.

En variante aux étapes 8 à 10, on peut utiliser des techniques permettant la détection directe des anticorps en tant que complexes avec l'antigène, telles que la réfractométrie, la diffraction de rayons lumineux par la surface réactionnelle, des méthodes de modification de la conductivité électrique ou du champs magnétique, etc.

L'originalité de la présente invention est qu'elle permet la mise au point d'un procédé capable de fixer et de mesurer tous les anticorps héparine-dépendants responsables de la TIH de type II qu'ils soient libres (Anti(Ag-SH)) ou complexés à l'antigène (SH-Ag-Anti(Ag-SH)), ceci grâce à l'utilisation d'une substance à forte affinité pour une susbtance héparinique, de préférence le sulfate de protamine ou la streptavidine (associée à l'héparine biotinylée), qui immobilise l'héparine sur la surface réactionnelle. En raison de sa grande disponibilité, de sa facilité d'obtention et de son faible coût, ce nouveau procédé (et ses variantes) permet d'augmenter la rapidité, la sensibilité et la reproductibilité du test et ainsi le diagnostic de patients à risque ou développant une thrombopénie induite par l'héparine.

Une substance à forte affinité pour une substance héparinique (SFA) est adsorbée ou liée par covalence à un support de réaction décrit plus bas. Cette substance à forte affinité pour une substance héparinique sert « d'ancrage » pour fixer l'héparine ou la substance héparinique et augmenter ainsi la surface réactionnelle du support. En particulier, on obtient un complexe à une concentration de 0,01 à 1000 µg/cm² de surface réactionnelle. La réaction suivante se produit :

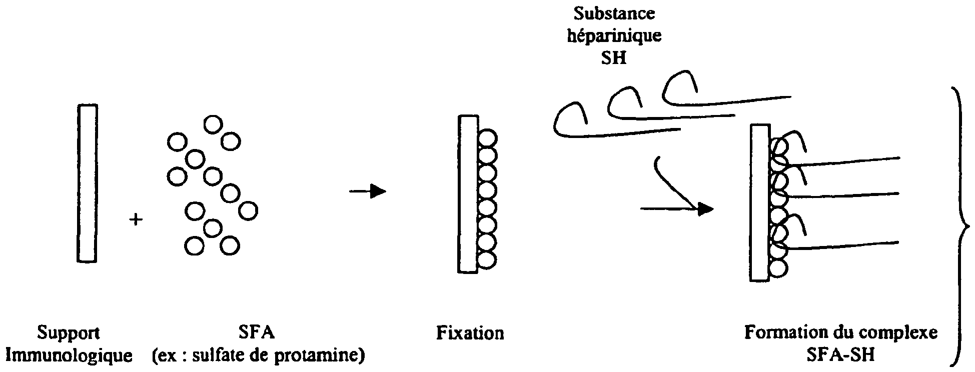

La substance à forte affinité pour la substance héparinique telle que le sulfate de protamine, la streptavidine (associée à une substance héparinique biotinylée), la poly-L-lysine, la poly-arginine, le polybrène, sert à fixer la substance héparinique selon la réaction (A) et par la suite cette substance héparinique fixera la substance antigénique issue éventuellement d'un lysat plaquettaire global si cette étape a été réalisée ou bien du sérum ou du plasma du patient à tester. Ce complexe substance antigénique-substance héparinique (Ag-SH) pourra ensuite être reconnu par les anticorps induits par la substance héparinique selon la réaction (B) (voir plus bas) si de tels anticorps sont présents dans le plasma ou le sérum du patient à tester. Ceci permettra pour finir de détecter les sujets à risque ou développant une TIH de type II grâce à la présente invention.

Dans un mode préféré de l'invention, on utilise le sulfate de protamine en tant que substance ayant une grande affinité pour la substance héparinique.

Dans un autre mode préféré, on utilise la streptavidine en tant que substance à forte affinité pour la substance héparinique (SFA), la substance héparinique étant sous forme biotinylée.

Dans encore un autre mode préféré de l'invention, on utilise la poly-L-lysine, la poly-arginine ou le polybrène en tant que substance ayant une grande affinité pour la substance héparinique.

Ce sulfate de protamine peut avoir diverses origines, il est généralement extrait du sperme de saumon. Pour être plus précis, la substance à forte affinité pour la substance héparinique est en fait ici utilisée en tant que support d'ancrage. La streptavidine est elle extraite de l'oeuf d'oiseaux, mais toute substance ayant les mêmes propriétés que le sulfate de protamine ou la streptavidine, et en particulier toute molécule recombinante ou de synthèse, peut être utilisée en lieu et place.

De manière avantageuse, dans la présente invention, la substance héparinique utilisée peut être de plusieurs natures et elle peut être représentée par :
- l'héparine non fractionnée (HNF) qui induit plus tôt et plus fréquemment l'apparition d'anticorps induits par l'héparine. Cette héparine a un poids moléculaire moyen de 6000-30000 daltons et un pouvoir rotatoire [α]_{D}²⁰ d'environ + 55° ;
- l'héparine de bas poids moléculaire ;
- des composés qui dérivent de l'héparine comme les héparinates métalliques (Ca²⁺ Li⁺, Na⁺, Mg²⁺, etc...) et des fragments de l'héparine ;
- des analogues de l'héparine comme les héparinoïdes (héparamine et ses sels, les chondroitines et leurs sels, etc...) ;
- les substances contenant l'héparine, ses dérivés et analogues comme les complexes de l'héparine et de ses dérivés ou analogues ;
- leurs mélanges
- l'une quelconque des molécules ci-dessus sous forme biotinylée.

Il est toutefois également possible d'utiliser dans la présente invention en tant que substance héparinique le polysulfate de pentosane, tout polysaccharide sulfaté chargé négativement ou des polymères de polystyrène sulfatés comme les polymères linéaires non-glycosaminoglycanes chargés négativement n'étant pas des hydrates de carbone, par exemple le polyvinylsulfate, le polyvinylsulfonate, le polystyrène sulfonate, le polyanétholsulfonate, des polyvinyl phosphate et polyvinylphosphonate, le poly-D glutamate, ou leurs formes biotinylées dans le cas de streptavidine en tant que substance à forte affinité pour la substance héparinique.

Il est à noter que l'origine, la longueur des chaînes polysaccharidiques et le degré de sulfatation des substances hépariniques semblent jouer un rôle important dans leur immunogénicité et donc dans le développement des TIH de type II. En effet, l'héparine d'origine bovine serait plus immunogène que l'héparine porcine. D'autre part, les héparines non fractionnées seraient également plus immunogènes (5 à 20 fois supérieure) que les héparines à bas poids moléculaires mais en raison de l'utilisation plus fréquente et prolongée de ces dernières, il a été décrit des TIH de type II induites par ces héparines. Les héparines non fractionnées sont capables de « déformer » davantage les molécules à forte affinité pour elle et génèrent des complexes de plus grande taille induisant une plus forte réponse immunitaire.

Les quantités relatives de substance à forte affinité pour la substance héparinique (SFA) et de substance héparinique (SH) sont : 1 mg de SFA pour 100 à 100000 UI d'héparine. De préférence, 1 mg de sulfate de protamine pour 100 à 1000 UI d'héparine (mais ces proportions peuvent varier en cas d'utilisation de variants du sulfate de protamine ou de l'héparine). Pour la streptavidine, on utilise 0,1 à 1000 µg de streptavidine pour 0.1 à 100 UI d'héparine biotinylée, et de préférence 1 à 50 µg de streptavidine pour 0,5 à 20 UI d'héparine, mais comme on est en excès, il n'y a pas de contrainte de limite.

La substance antigénique potentielle ou Ag est constituée par une substance ayant une forte affinité pour l'héparine ou une substance héparinique décrites ci-dessus. Elle provient du plasma ou du sérum du patient à tester ou bien optionnellement si cela est nécessaire d'un lysat plaquettaire global. Cette substance antigénique réagira aisément avec ladite substance héparinique (fixée au préalable à la substance à forte affinité pour l'héparine, SFA, le plus souvent du sulfate de protamine ou de la streptavidine, la substance héparinique étant biotinylée) et formera ainsi un complexe avec elle, déterminé par l'abréviation (Ag-SH).

Pour qu'il n'y ait pas de confusion ultérieurement, il convient de préciser cette dénomination :
La substance antigénique (par exemple un facteur issu des plaquettes) est appelée ainsi non parce qu'elle induit à elle seule une production d'anticorps, une réponse immunitaire mais parce qu'une fois liée à la substance héparinique pour laquelle elle a une forte affinité, elle devient immunogène. Ainsi le complexe formé substance antigénique-substance héparinique (Ag-SH) pourra ensuite être reconnu par les anticorps induits par l'héparine et contenus potentiellement dans le plasma et le sérum du patient à tester. Après une révélation adaptée, il sera possible ainsi de détecter les sujets à risque ou développant une TIH de type II grâce à la présente invention et selon les réactions suivantes :

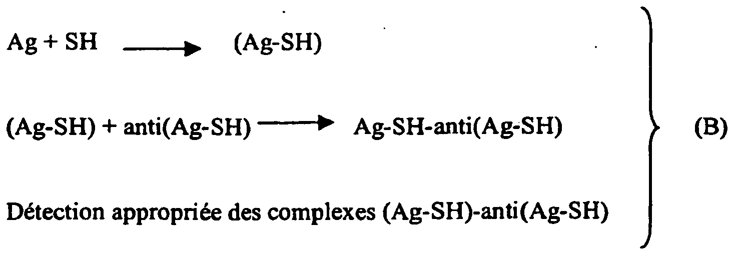

Pour la détermination et le diagnostic des pathologies immunes ou auto-immunes potentialisées par une substance héparinique telle que la thrombopénie induite par l'héparine (TIH de type II) selon l'invention, ladite substance antigénique sera choisie parmi l'ensemble des molécules ou des complexes ayant une forte affinité pour une substance héparinique. Ces substances antigéniques sont issues du plasma ou du sérum du patient ou alors d'un lysat plaquettaire global ou d'un lysat leucocytaire global ou de tout milieu biologique la contenant. Elles peuvent être :
- le facteur plaquettaire 4 ou pF4
- des fractions de ce facteur pF4
- des fractions contenant au moins une substance éluée en même temps que le pF4
- le pF4 recombinant et ses variants
- des peptides synthétiques reprenant toute ou partie de la séquence des aminoacides du pF4
- le protéoglycane
- des complexes protéoglycane-pF4
- ainsi que leurs mélanges.
- la Béta-Thromboglobuline
- le NAP2
- les PDGF
- des glycoprotéines plaquettaires
- l'Interleukine 8 (IL-8),
- le FGF (fibroblast growth factors), de forte affinité pour l'héparine,
- etc.......

Auparavant, la substance antigénique était principalement du pF4, obtenu à partir d'un lysat plaquettaire purifié, ou de pF4 recombinant fonctionnel et purifié, et servait de cible antigénique car une fois complexé à la substance héparinique, il induisait la réaction d'anticorps héparine-dépendants. Dans la présente invention, la substance héparinique sera fixée au support réactionnel défini plus bas via une substance ayant une très grande affinité pour elle (SFA, par exemple, le sulfate de protamine, la streptavidine, la poly-L-lysine, la poly-arginine, le polybrène) augmentant ainsi la surface réactionnelle du support de réaction. La substance antigénique reconnue par l'anticorps pourra être du pF4 car un lysat plaquettaire global, contenant entre autres du pF4, sera ajouté au complexe SFA-substance héparinique (par exemple, sulfate de protamine-héparine). Mais tous les anticorps héparine-dépendants anti(Ag-SH) pourront être détectés et non seulement ceux qui dépendent du pF4. L'un des avantages majeurs de la présente invention réside dans le fait que ces différentes substances sont utilisées dans la présente invention sans nécessiter de purification, sans purification au préalable de la substance antigénique réagissant avec la substance héparinique.

Le double avantage de ce procédé est d'une part une réduction du coût de mise en oeuvre de la présente invention car aucune étape de purification n'est nécessaire et d'autre part une sensibilité et une détection accrue par fixation de l'héparine biologiquement disponible qui permet la fixation des Ag mais aussi des Ag déjà fixés à leur anticorps et formant déjà des complexes (Ag-SH-Anti(Ag-SH)). Par exemple, l'héparine fixée en grand nombre au support, grâce à la SFA, pourra à son tour fixer par exemple le pF4 seul mais également le pF4 déjà fixé à son anticorps présent dans le plasma ou le sérum du patient. La sensibilité et la détection seront alors accrues avec un tel procédé.

Les tests peuvent être sensibilisés éventuellement avec un lysat plaquettaire global provenant d'un concentré plaquettaire préparé à partir des plaquettes de donneurs normaux et obtenu par des techniques classiques connues de l'homme du métier. Ce lysat plaquettaire apporte des protéines plaquettaires en excès de concentration, ce qui permet de constituer la cible antigénique des anticorps héparine-dépendants sur la substance à forte affinité pour la substance héparinique (SFA), en présence d'un excès de substance héparinique. Cette sensibilisation est due au fait que le plasma ou le sérum à tester peut contenir des quantités variables de protéines plaquettaires et parfois en quantité insuffisantes pour une bonne réaction. Le lysat plaquettaire les apporte en quantité suffisante.

La quantité de lysat plaquettaire global utilisée peut aller de quelques µl à 500 µl ou plus. En règle générale, d'excellents résultats sont obtenus avec un lysat plaquettaire dilué du 1 :10 au 1 :1000, et de préférence du 1 :10 au 1 :100, dans le milieu réactionnel. Toutefois, l'utilisation de très faibles quantités (lysat dilué au 1 :100000 ou plus) est possible pour un diagnostic fiable.

Pour faire un diagnostic ciblé, sur le mélange réactionnel lavé, on ajoutera le sérum ou le plasma du patient susceptible d'avoir développé des anticorps héparine-dépendants. Si ce sérum ou ce plasma contient des anticorps induits par l'héparine, la réaction suivante se produira :

(Ag-SH) + sérum ou plasma du patient à tester → (Ag-SH)-anti(Ag-SH)

Ce dernier complexe sera révélé par une technique connue et appropriée.

Le plasma du patient (ou le sérum) est testé habituellement à une dilution comprise entre 1 :1 et 1 :500, mais d'autres variantes sont possibles au-delà de ces bornes. Par exemple, pour la technologie utilisant des particules de latex, le sérum ou plasma sont utilisés purs. Le volume réactionnel va de quelques µl à 0.5 ml. On va utiliser en règle générale de moins de 1 µl à 200 µl de sérum ou plasma de patient.

De manière avantageuse, la révélation du complexe résultant Ag-SH-anti(Ag-SH) peut être réalisée par des tests EIA (tests enzymo-immunologiques), classiquement des tests ELISA, ou par RIA (tests radio-immunologiques) mais également par immunofluorescence, par immuno-turbidimétrie, par agglutination de latex sur lame, par néphélométrie, par turbidimétrie ou néphélométrie amplifiée par des particules de latex ou par toute autre technique classique ou nouvelle, sensible et connue pour la détection d'anticorps, ou de la formation de complexes antigène-anticorps.

Les méthodes d'immunochimie de détection utilisent généralement une marque enzymatique (peroxydase, glucose oxydase, phosphatase alcaline). La réaction immunologique est suivie par une réaction indicatrice qui permet la détection photométrique de l'activité enzymatique liée au complexe (Ag-SH)-anti(Ag-SH). Le plus souvent c'est un deuxième anticorps anti-immunoglobuline (anti-Ig) qui est marqué par une enzyme * , et la réaction se développe en phase solide selon un principe non compétitif :

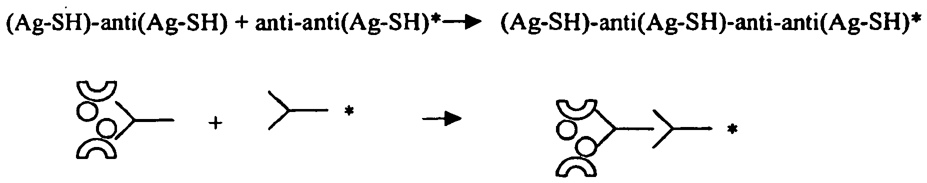

Cette enzyme * catalyse l'oxydation d'un substrat chromogène et l'intensité de la coloration est proportionnelle à la concentration de l'anticorps induit par l'héparine à mesurer dans le plasma ou le sérum du patient. Le plus souvent l'enzyme utilisée est la peroxydase et son substrat chromogène est l'OPD ou ortho-phénylènediamine, et de plus en plus souvent le TMB (Tétra-Methyl-Benzidine). Suivant la technique utilisée, le complexe Ag-SH-anti(Ag-SH) pourra également être révélé par un moyen fluorogène, un radio-isotope, une particule de latex colorée, de l'or colloïdal, etc...

Les anticorps anti(Ag-SH) issus du plasma du patient et les deuxièmes anticorps anti-Ig peuvent être des anticorps polyclonaux ou monoclonaux (IgG, IgA ou IgM).

Alternativement, les anticorps peuvent être détectés directement en tant que complexes avec l'antigène à l'aide des nouvelles méthodes de détection comme la réfractométrie, la diffraction de rayons lumineux par la surface réactionnelle, des méthodes de modification de la conductivité électrique ou du champ magnétique, etc...

Pour la révélation du complexe (Ag-SH)-Anti(Ag-SH), il n'y a pas de contrainte de concentration particulière. On utilise des concentrations de 0,01 µg à 1 mg mais en règle générale, on utilise des concentrations de 0,1 à 100 µg.

Les supports de réactions immunologiques utilisés pour ces tests de détection des anticorps héparine-dépendants et de diagnostic des TIH de type II selon la présente invention peuvent être de différentes natures et sont choisis parmi :
les lames, les micro-plaques, les latex fluorescents, les latex magnétiques, les membranes d'immuno-filtration ou les membranes d'immuno-migration, les biochips, les billes, les ailettes, les tubes, mais également les liposomes, les vésicules lipidiques, les micro-particules biologiques ou obtenues à partir de polymères, ou les émulsions etc...

De préférence, on utilisera des billes de latex.

Le complexe substance à forte affinité pour la substance héparinique-substance héparinique (soit SFA-SH) est fixé au support de réactions immunologiques par adsorption ou par liaison covalente.

Dans une variante de la présente invention, on utilisera deux substances à forte affinité pour l'héparine telles que par exemple le sulfate de protamine combiné au polybrène ou le sulfate de protamine combiné à la poly-L-lysine.

Une combinaison de au moins deux substances à forte affinité pour l'héparine pourront être réalisées en choisissant ces substances parmi le sulfate de protamine, la poly-L-lysine, la poly-arginine, le polybrène ou la streptavidine, la substance héparinique étant dans ce dernier cas biotinylée.

Dans une autre variante de la présente invention, on utilisera deux substances hépariniques telles que par exemple l'héparine non fractionnée combinée au polysulfate de pentosane, une héparine combinée au polyvinylsulfate.

Une combinaison de au moins deux substances hépariniques pourront être réalisées en choisissant ces substances parmi celles citées dans la présente description.

Dans encore une autre variante, on utilisera deux substances antigéniques telles que par exemple le pF4 et l'interleukine 8 (IL-8).

Une combinaison de au moins deux substances antigéniques pourront être réalisées en choisissant ces substances parmi celles citées précédemment.

Ces exemples de combinaisons ne sont bien évidemment pas limitatifs, il pourra être utilisé d'autres combinaisons de plusieurs substances à forte affinité pour l'héparine, d'autres combinaisons de plusieurs substances hépariniques et d'autres combinaisons de plusieurs substances antigéniques. Des mélanges de ces différentes combinaisons peuvent également être utilisés. En effet, la présente invention concerne un procédé consistant en partie à faire réagir au moins une substance à forte affinité pour une substance héparinique (SFA) avec au moins une substance héparinique (SH) puis avec au moins une substance antigénique. Chacune des différentes combinaisons utilisées donnent des résultats similaires à ceux donnés dans les exemples ci-dessous.

La présente invention concerne également un nécessaire mettant en oeuvre le procédé de la présente invention tel que décrit précédemment, selon la revendication 7.

Les caractéristiques de ce procédé et de ce nécessaire de détection des anticorps induits par héparine et de diagnostic des pathologies immunes ou auto-immunes potentialisées par une substance héparinique telle que la thrombopénie induite par l'héparine seront mieux comprises et illustrées à la lecture des exemples de réalisation qui suivent :

### Exemples de réalisations du test pour le diagnostic rapide des thrombopénies induites_par l'héparine (TIH)

### Exemple 1 :

Une plaque Elisa (exemple Maxisorb Type 1 avec certificat de NUNC ou Covalink de NUNC) est incubée avec 200 µl (50 - 250 possibles) par puits d'un mélange de sulfate de protamine (extrait du sperme de Saumon et fourni par Sigma) à 10 µg/ml et 20 UI/ml d'héparine non fractionnée (type calciparine de Choay/Sanofi-Aventis) en tampon phosphate 0.05 M à pH 7.50 ou carbonate 0.05 M à pH 9.6, pendant 16 à 24 heures à TA ou à + 4°C. Après lavage, la plaque est saturée par du sérum animal (chèvre, boeuf, mouton ou autre) à 10-20 % en tampon phosphate 0.05 M à pH 7.50. Après incubation de 1 à 48 heures à TA ou à + 4°C, la plaque est lavée et utilisée immédiatement, ou stabilisée pour utilisation ultérieure.

Lors du test, 200 µl du plasma ou sérum à tester dilués au 1 :100 en tampon phosphate 0.05 M, 0.15 M de chlorure de sodium et 10 à 20 % de sérum animal (chèvre, boeuf, mouton ou autre), sont introduits dans les puits et incubés 1 à 2 heures à TA. Après cette incubation, les plaques sont lavées et le révélateur, anticorps spécifique des immunoglobulines humaines IgG, ou IgM ou IgA couplés à la péroxydase, utilisés à 0.1 à 5 µg/ml en tampon phosphate 0.05 M, NaCl 0.15 M, 1 % d'albumine sérique bovine et à pH 7.50, (ou à la phosphatase alcaline) sont introduits et incubés pendant 1 à 2 heures à TA. Après un nouveau lavage, le substrat est introduit (OPD/H2O2 ou TMB/H2O2 pour la péroxydase ou PNP pour la phosphatase alcaline). Et incubés à TA. Après 2 à 30 minutes d'incubation (selon la variante utilisée) la réaction est arrêtée par l'acide sulfurique (péroxydase), de 0.25 à 2 M ou l'hydroxyde de sodium à une concentration de 0.1 à 2 M (pour la phosphatase alcaline) et la DO est lue à 492 nm (OPD), ou à 450 nm (TMB) ou à 405 nm (PNP). La présence d'anticorps associés au risque ou à la pathologie de TIH se manifeste par des DO élevées, supérieures à 0.30 et le plus souvent très élevées, généralement au-dessus de 1.00. Les plasmas normaux ont en général une DO inférieure à 0.20.

Dans une variante avantageuse de la technique, le lysat plaquettaire est ajouté dans les puits (50 µl d'une dilution de 1 :10 à 1 :1000 en tampon phosphate 0.05 M, NaCl 0.15 M à pH 7.50), ou inclus dans le diluant de l'échantillon (sérum ou plasma du malade) à une dilution allant du 1 :10 au 1 :1000. Le protocole est ensuite continué comme indiqué.

### Exemple 2 :

Une plaque Elisa (exemple Maxisorb Type 1 avec certificat de NUNC ou Covalink de NUNC) est incubée avec 200 µl (50 - 250 possibles) par puits de streptavidine (Roche, Mannheim, Allemagne) à 5 µg/ml en tampon carbonate 0.05 M à pH 9.60. Après incubation de 12 à 48 heures à TA ou à °C et lavage, 200 µl par puits d'héparine biotinylée (Sigma) à une concentration de 1 à 25 UI/ml sont ajoutés et incubés 1 à 24 heures à TA ou à + 4°C. Après lavage, la plaque est saturée par du sérum animal (chèvre, boeuf, mouton ou autre) à 10-20 % en tampon phosphate 0.05 M à pH 7.50. Alternativement, des plaques streptavidine du commerce (Roche, Nunc, Greiner, etc...) peuvent être utilisées pour fixation de l'héparine. Après incubation de 1 à 48 heures à TA ou à + 4°C, la plaque est lavée et utilisée immédiatement, ou stabilisée pour utilisation ultérieure. Lors du test, 200 µl du plasma ou sérum à tester dilués au 1 :100 en tampon phosphate 0.05 M, 0.15 M de chlorure de sodium et 10 à 20 % de sérum animal (chèvre, boeuf, mouton ou autre), sont introduits dans les puits et incubés 1 à 2 heures à TA. Après cette incubation, les plaques sont lavées et le révélateur, anticorps spécifique des immunoglobulines humaines IgG, ou IgM ou IgA couplés à la péroxydase, utilisés à une concentration de 0.1 à 5 µg/ml en tampon phosphate 0.05 M, NaCl 0.15 M, 1 % d'albumine sérique bovine et à pH 7.50, (ou à la phosphatase alcaline) sont introduits et incubés pendant 1 à 2 heures à TA. Après un nouveau lavage, le substrat est introduit, (OPD/H2O2 ou TMB/H2O2 pour la péroxydase ou PNP pour la phosphatase alcaline). et incubés à TA. Après 2 à 30 minutes d'incubation (selon la variante utilisée) la réaction est arrêtée par l'acide sulfurique (péroxydase) à 0.25 à 2 M ou l'hydroxyde de sodium à 0.1 à 2 M (pour la phosphatase alcaline) et la DO est lue à 492 nm (OPD), ou à 450 nm (TMB) ou à 405 nm (PNP). La présence d'anticorps associés au risque ou à la pathologie de TIH se manifeste par des DO élevées, supérieures à 0.30 et le plus souvent très élevées, généralement au-dessus de 1.00. Les plasmas normaux ont en général une DO inférieure à 0.20.

Dans une variante avantageuse de la technique, le lysat plaquettaire est ajouté dans les puits (50 µl d'une dilution de 1 :10 à 1 :1000 en tampon phosphate 0.05 M, NaCl 0.15 M à pH 7.50), ou inclus dans le diluant de l'échantillon (sérum ou plasma du malade) à une dilution allant du 1 :10 au 1 :1000. Le protocole est ensuite continué comme indiqué.

### Exemple 3 :

Des billes de latex en polystyrène ou tout autre polymère, de taille comprise entre 20 et 2000 nm, (polybutyl-méthacrylate) non fonctionnalisées ou dérivatisées (COOH, ou NH2, ou SH, ou CHCl2, ou OH etc...) sont couplées avec du sulfate de protamine à raison de 0.01 à 10 mg par 100 mg de billes de latex (soit par ml de suspension colloïdale de latex à 10 % ou par 2 ml de suspension colloïdale à 5 %) par simple adsorption ou par liaison covalente selon les techniques connues de l'homme de l'art et disponibles dans la littérature. L'héparine est ajoutée à une concentration comprise entre 1 et 1000 UI/ml. Après lavage, dialyse ou tout autre traitement approprié pour enlever l'excès d'héparine,, les particules en suspension colloïdale sont stabilisées, et amenées à 0.5 % en tampon phosphate ou glycine ou borate à 0.05 M, contenant 0.15 M de chlorure de sodium, 1 % d'Albumine sérique bovine, du Poly-Ethylène-Glycol, et éventuellement un surfactant comme le tween 20 ou 80 ou le SDS (0.01 à 0.1 %).

Ces particules peuvent être utilisées pour des tests d'agglutination sur lame ou pour des tests photométriques ou néphélométriques.

Dans la variante où le lysat plaquettaire est utilisé pour sensibiliser la technique, celui-ci est ajouté à une concentration du 1 :2 au 1 :200 dans le diluant de l'échantillon à tester (plasma ou sérum du malade) ou directement dans la solution mère de la suspension colloïdale de latex.

Pour des tests sur lame, sur une lame en verre 20 à 50 µl de plasma ou sérum à tester, non dilué ou dilué du 1 :2 au 1 :10 sont incubés avec 20 à 50 µl de particules de latex sensibilisés par le sulfate de protamine et l'héparine et stabilisées. Après mélange et agitation, l'apparition d'une agglutination est observée. Elle indique la présence d'anticorps.

Une autre utilisation des billes consiste dans les tests immuno-turbidimètriques ou néphélométriques. Les billes sont amenées à 0.1 % de concentration en tampon phosphate 0.05 M, contenant 0.15 M de chlorure de sodium et 1 % d'albumine sérique bovine. Dans le test 250 µl de billes sont incubées avec 250 µl de sérum ou plasma à tester dilué du 1 :1 au 1 :100 dans un tampon phosphate 0.05 M, contenant 0.15 M de chlorure de sodium, 1 % d'albumine sérique bovine et de 0.1 à 10 % de polyéthylène glycol, à un pH de 7.00 à 9.50. Après 1 à 60 min d'incubation la variation de DO est mesurée à une longueur d'onde pouvant aller de 300 à 1000 nm. Si les anticorps sont présents, l'augmentation de l'absorbance sur 60 min est supérieure à 0.100 et le plus souvent comprise entre 0.200 et 2.00. Cela indique la présence d'anticorps héparine dépendants pouvant être impliqués dans les TIH. La réaction peut être sensibilisée en utilisant un polymère comme le Poly-Ethylène-Glycol (PEG) ou la Poly-Vinyl-Pirolydone (PVP) dans le tampon de réaction. Dans la variante où le lysat plaquettaire est utilisé pour sensibiliser la technique, celui-ci est ajouté à une concentration du 1 :2 au 1 :200 dans le diluant de l'échantillon à tester (plasma ou sérum du malade) ou directement dans la solution mère de la suspension colloïdale de latex.

### Exemple 4 :

Des billes de latex en polystyrène ou tout autre polymère (polybutyl-méthacrylate), de taille comprise entre 20 et 2000 nm, non fonctionnalisées ou dérivatisées (COOH, ou NH2, ou SH, ou CHCl2, ou OH etc...) sont couplées avec de la streptavidine (Roche, Mannheim, Allemagne) à raison de 0.01 à 10 mg par 100 mg de billes de latex (soit par ml de suspension colloïdale de latex à 10 % ou par 2 ml de suspension colloïdale à 5 %) par simple adsorption ou par liaison covalente selon les techniques connues de l'homme de l'art et disponibles dans la littérature. L'héparine biotinylée (Sigma) est ensuite ajoutée à raison de 0.05 à 50 UI/ml, et incubée avec les particules de latex pendant 1 à 24 heures à TA ou à + 4 °C.

Après lavage, dialyse ou tout autre traitement approprié afin d'enlever l'excès d'héparine, les particules en suspension colloïdale sont stabilisées, et amenées à 0.5 % en tampon phosphate ou glycine ou borate à 0.05 M, contenant 0.15 M de chlorure de sodium, 1 % d'Albumine sérique bovine, du Poly-Ethylène-Glycol, et éventuellement un surfactant comme le tween 20 ou 80 ou le SDS (0.01 à 0.1 %).

Ces particules peuvent être utilisées pour des tests d'agglutination sur lame ou pour des tests photométriques ou néphélométriques.

Pour des tests sur lame, sur une lame en verre 20 à 50 µl de plasma ou sérum à tester, non dilué ou dilué du 1 :2 au 1 :10 sont incubés avec 20 à 50 µl de particules de latex sensibilisés par la streptavidine et l'héparine biotinylée et stabilisées. Après mélange et agitation, l'apparition d'une agglutination est observée. Elle indique la présence d'anticorps.

Dans la variante où le lysat plaquettaire est utilisé pour sensibiliser la technique, celui-ci est ajouté à une concentration du 1 :2 au 1 :200 dans le diluant de l'échantillon à tester (plasma ou sérum du malade) ou directement dans la solution mère de la suspension colloïdale de latex.

Une autre utilisation des billes consiste dans les tests immuno-turbidimètriques ou néphélométriques. Les billes sont amenées à 0.1 % de concentration en tampon phosphate 0.05 M, contenant 0.15 M de chlorure de sodium et 1 % d'albumine sérique bovine. Dans le test 250 µl de billes sont incubées avec 250 µl de sérum ou plasma à tester dilué du 1 :1 au 1 :100 dans un tampon phosphate 0.05 M, contenant 0.15 M de chlorure de sodium, 1 % d'albumine sérique bovine et de 0.1 à 10 % de polyéthylène glycol, à un pH de 7.00 à 9.50. Après 1 à 60 min d'incubation la variation de DO est mesurée à une longueur d'onde pouvant aller de 300 à 1000 nm. Si les anticorps sont présents, l'augmentation de l'absorbance sur 60 min est supérieure à 0.100 et le plus souvent comprise entre 0.200 et 2.00. Cela indique la présence d'anticorps héparine dépendants pouvant être impliqués dans les TIH. La réaction peut être sensibilisée en utilisant un polymère comme le Poly-Ethylène-Glycol (PEG) ou la Poly-Vinyl-Pirolydone (PVP) dans le tampon de réaction.

Dans la variante où le lysat plaquettaire est utilisé pour sensibiliser la technique, celui-ci est ajouté à une concentration du 1 :2 au 1 :200 dans le diluant de l'échantillon à tester (plasma ou sérum du malade) ou directement dans la solution mère de la suspension colloïdale de latex.

### Exemple 5

Une plaque Elisa (exemple Maxisorb Type 1 avec certificat de NUNC ou Covalink de NUNC) est incubée avec 200 µl (50 - 250 possibles) par puits d'un mélange de sulfate de protamine (extrait du sperme de Saumon et fourni par Sigma) et de poly-L-lysine entre 10 à 20 µg/ml et de 10 à 20 UI/ml d'héparine non fractionnée (type calciparine de Choay/Sanofi-Aventis) et de 50 à 100 µg de polysulfate de pentosane en tampon phosphate 0.05 M à pH 7.50 ou carbonate 0.05 M à pH 9.6, pendant 16 à 24 heures à TA ou à + 4°C. Après lavage, la plaque est saturée par du sérum animal (chèvre, boeuf, mouton ou autre) à 10-20 % en tampon phosphate 0.05 M à pH 7.50. Après incubation de 1 à 48 heures à TA ou à + 4°C, la plaque est lavée et utilisée immédiatement, ou stabilisée pour utilisation ultérieure.

Lors du test, 200 µl du plasma ou sérum à tester dilués au 1 :100 en tampon phosphate 0.05 M, 0.15 M de chlorure de sodium et 10 à 20 % de sérum animal (chèvre, boeuf, mouton ou autre), sont introduits dans les puits et incubés 1 à 2 heures à TA. Après cette incubation, les plaques sont lavées et le révélateur, anticorps spécifique des immunoglobulines humaines IgG, ou IgM ou IgA couplés à la péroxydase, utilisés à 0.1 à 5 µg/ml en tampon phosphate 0.05 M, NaCl 0.15 M, 1 % d'albumine sérique bovine et à pH 7.50, (ou à la phosphatase alcaline) sont introduits et incubés pendant 1 à 2 heures à TA. Après un nouveau lavage, le substrat est introduit (OPD/H2O2 ou TMB/H2O2 pour la péroxydase ou PNP pour la phosphatase alcaline). Et incubés à TA. Après 2 à 30 minutes d'incubation (selon la variante utilisée) la réaction est arrêtée par l'acide sulfurique (péroxydase), de 0.25 à 2 M ou l'hydroxyde de sodium à une concentration de 0.1 à 2 M (pour la phosphatase alcaline) et la DO est lue à 492 nm (OPD), ou à 450 nm (TMB) ou à 405 nm (PNP). La présence d'anticorps associés au risque ou à la pathologie de TIH se manifeste par des DO élevées, supérieures à 0.30 et le plus souvent très élevées, généralement au-dessus de 1.00. Les plasmas normaux ont en général une DO inférieure à 0.20.

Dans une variante avantageuse de la technique, le lysat plaquettaire est ajouté dans les puits (50 µl d'une dilution de 1 :10 à 1 :1000 en tampon phosphate 0.05 M, NaCl 0.15 M à pH 7.50), ou inclus dans le diluant de l'échantillon (sérum ou plasma du malade) à une dilution allant du 1 :10 au 1 :1000. Le protocole est ensuite continué comme indiqué

Dans une autre variante de la technique, le lysat plaquettaire (25 µl d'une dilution de 1 :10 à 1 :1000 en tampon phosphate 0.05 M, NaCl 0.15 M à pH 7.50) et l'IL8 recombinante (25 µl d'une solution diluée entre 1 et 20µg/ml en tampon phosphate 0.05 M, NaCl 0.15 M à pH 7.50) sont ajoutés dans les puits, ou inclus dans le diluant de l'échantillon (sérum ou plasma du malade) à une dilution allant du 1 :10 au 1 :1000 pour le lysat plaquettaire et entre 1 et 20 µg/ml pour l'IL8 recombinante. Le protocole est ensuite continué comme indiqué.

Dans une autre variante de la technique, le lysat plaquettaire (25 µl d'une dilution de 1 :10 à 1 :1000 en tampon phosphate 0.05 M, NaCl 0.15 M à pH 7.50) et le lysat leucocytaire (25 µl d'une dilution de 1 :10 à 1 :1000 en tampon phosphate 0.05 M, NaCl 0.15 M à pH 7.50) sont ajoutés dans les puits, ou inclus dans le diluant de l'échantillon (sérum ou plasma du malade) à une dilution allant du 1 :10 au 1 :1000 pour le lysat plaquettaire ainsi que le lysat leucocytaire. Le protocole est ensuite continué comme indiqué.

### Exemple 6

Une plaque Elisa (exemple Maxisorb Type 1 avec certificat de NUNC ou Covalink de NUNC) est incubée avec 200 µl (50 - 250 possibles) par puits de streptavidine (Roche, Mannheim, Allemagne) à 5 µg/ml en tampon carbonate 0.05 M à pH 9.60. Après incubation de 12 à 48 heures à TA ou à °C et lavage, 200 µl par puits d'héparine biotinylée (Sigma) à une concentration de 1 à 25 UI/ml sont ajoutés et incubés 1 à 24 heures à TA ou à + 4°C. Après lavage, la plaque est saturée par du sérum animal (chèvre, boeuf, mouton ou autre) à 10-20 % en tampon phosphate 0.05 M à pH 7.50. Alternativement, des plaques streptavidine du commerce (Roche, Nunc, Greiner, etc...) peuvent être utilisées pour fixation de l'héparine. Après incubation de 1 à 48 heures à TA ou à + 4°C, la plaque est lavée et utilisée immédiatement, ou stabilisée pour utilisation ultérieure. Lors du test, 200 µl du plasma ou sérum à tester dilués au 1 :100 en tampon phosphate 0.05 M, 0.15 M de chlorure de sodium et 10 à 20 % de sérum animal (chèvre, boeuf, mouton ou autre), sont introduits dans les puits et incubés 1 à 2 heures à TA. Après cette incubation, les plaques sont lavées et le révélateur, anticorps spécifique des immunoglobulines humaines IgG, ou IgM ou IgA couplés à la péroxydase, utilisés à une concentration de 0.1 à 5 µg/ml en tampon phosphate 0.05 M, NaCl 0.15 M, 1 % d'albumine sérique bovine et à pH 7.50, (ou à la phosphatase alcaline) sont introduits et incubés pendant 1 à 2 heures à TA. Après un nouveau lavage, le substrat est introduit, (OPD/H2O2 ou TMB/H2O2 pour la péroxydase ou PNP pour la phosphatase alcaline). et incubés à TA. Après 2 à 30 minutes d'incubation (selon la variante utilisée) la réaction est arrêtée par l'acide sulfurique (péroxydase) à 0.25 à 2 M ou l'hydroxyde de sodium à 0.1 à 2 M (pour la phosphatase alcaline) et la DO est lue à 492 nm (OPD), ou à 450 nm (TMB) ou à 405 nm (PNP). La présence d'anticorps associés au risque ou à la pathologie de TIH se manifeste par des DO élevées, supérieures à 0.30 et le plus souvent très élevées, généralement au-dessus de 1.00. Les plasmas normaux ont en général une DO inférieure à 0.20.

Dans une variante avantageuse de la technique, le lysat plaquettaire (25 µl d'une dilution de 1 :10 à 1 :1000 en tampon phosphate 0.05 M, NaCl 0.15 M à pH 7.50) et l'IL8 recombinante (25 µl d'une solution diluée entre 1 et 20µg/ml en tampon phosphate 0.05 M, NaCl 0.15 M à pH 7.50) sont ajoutés dans les puits, ou inclus dans le diluant de l'échantillon (sérum ou plasma du malade) à une dilution allant du 1 :10 au 1 :1000 pour le lysat plaquettaire et entre 1 et 20 µg/ml pour 1'IL8 recombinante. Le protocole est ensuite continué comme indiqué.

Dans une autre variante de la technique, le lysat plaquettaire (25 µl d'une dilution de 1 :10 à 1 :1000 en tampon phosphate 0.05 M, NaCl 0.15 M à pH 7.50) et le lysat leucocytaire (25 µ%l d'une dilution de 1 :10 à 1 :1000 en tampon phosphate 0.05 M, NaCl 0.15 M à pH 7.50) sont ajoutés dans les puits, ou inclus dans le diluant de l'échantillon (sérum ou plasma du malade) à une dilution allant du 1 :10 au 1 :1000 pour le lysat plaquettaire ainsi que le lysat leucocytaire. Le protocole est ensuite continué comme indiqué.

## Revendications

1. **Procédé** pour la détection des anticorps héparine-dépendants et le diagnostic des pathologies immunes ou auto-immunes potentialisées par une substance héparinique choisies parmi la thrombopénie produite par la présence de polysulfate de pentosane ou la thrombopénie induite par l'héparine (TIH de type II) en tant que médicament inducteur, ledit procédé étant **caractérisé en ce qu'**il comprend les étapes consistant à :
1) à faire réagir :
- au moins une substance à forte affinité pour une substance héparinique (SFA) immobilisée sur un support solide, constituée par toute molécule ou complexe ayant une forte affinité pour une substance héparinique,
- avec au moins une substance héparinique (SH) choisie parmi l'héparine, les dérivés hépariniques, les polymères linéaires non-glycosaminoglycan chargés négativement n'étant pas un hydrate de carbone choisis parmi le polyvinylsulfate, le polyvinylsulfonate,
le polystyrène sulfonate, le polyanétholsulfonate, des polyvinyl phosphate et polyvinylphosphonate, le poly-D glutamate, pour former un complexe substance à forte affinité pour la substance héparinique-substance héparinique (SFA-SH), la substance héparinique étant en léger ou large excès, afin de pouvoir fixer d'autres substances affines pour une substance héparinique,
- puis après au moins un lavage pour éliminer la au moins une substance héparinique non fixée,
- avec un lysat plaquettaire consistant en un concentré de plaquettes normales ou lysées et/ ou un lysat leucocytaire, contenant des molécules affines pour la substance héparinique (SH) potentiellement antigéniques et appelées substances antigéniques potentielles (Ag), sans purification préalable desdites substances antigéniques potentielles et capables de réagir avec la substance héparinique, formant ainsi un complexe (Ag-SH),
- et enfin avec le plasma ou le sérum d'un patient à tester (dilué, de préférence à une dilution de l'ordre de 1 :100) contenant potentiellement :
un matériau anticorps anti(Ag-SH) généré dans l'organisme après administration d'une substance héparinique et qui reconnaît (Ag-SH), selon la réaction :
(Ag-SH) + anti(Ag-SH) --> (Ag-SH)-anti(Ag-SH)
et/ou au moins un complexe immun constitué d'un complexe Ag-SH-anticorps anti(Ag-SH) présent dans le plasma ou sérum du patient capables de réagir avec le complexe substance à forte affinité pour la substance héparinique-substance héparinique (SFA-SH), puis,
2) révéler le complexe produit (Ag-SH)-anti(Ag-SH) résultant après au moins un lavage pour éliminer toute substance pouvant interférer avec le système de détection.

2. **Procédé** pour la détection des anticorps héparine-dépendants et le diagnostic des pathologies immunes ou auto-immunes potentialisées par une substance héparinique choisies parmi la thrombopénie produite par la présence de polysulfate de pentosane ou la thrombopénie induite par l'héparine (TIH de type II) en tant que médicament inducteur, ledit procédé étant **caractérisé en ce qu'**il consiste en les étapes suivantes :
1) à faire réagir :
- au moins une substance à forte affinité pour une substance héparinique (SFA) immobilisée sur un support solide et choisie parmi le sulfate de protamine, la poly-L-lysine, la poly-arginine, le polybrène ou la streptavidine, la au moins une substance héparinique étant dans ce dernier cas biotinylée,
- avec au moins une substance héparinique (SH) choisie parmi l'héparine, les dérivés hépariniques, les polymères linéaires non-glycosaminoglycan chargés négativement n'étant pas un hydrate de carbone choisis parmi le polyvinylsulfate, le polyvinylsulfonate, le polystyrène sulfonate, le polyanétholsulfonate, des polyvinyl phosphate et polyvinylphosphonate, le poly-D glutamate, pour former un complexe substance à forte affinité pour la substance héparinique-substance héparinique (SFA-SH), la substance héparinique étant en léger ou large excès, afin de pouvoir fixer d'autres substances affines pour une substance héparinique,
- puis après au moins un lavage pour éliminer la au moins une substance héparinique non fixée,
- enfin avec le plasma ou le sérum d'un patient à tester (dilué, de préférence à une dilution de l'ordre de 1 :100) contenant potentiellement :
un matériau anticorps anti(Ag-SH) généré dans l'organisme après administration d'une substance héparinique et qui reconnaît (Ag-SH), selon la réaction :
(Ag-SH) + anti(Ag-SH) --> (Ag-SH)-anti(Ag-SH)
et/ou au moins un complexe immun constitué d'un complexe Ag-SH-anticorps anti(Ag-SH) présent dans le plasma ou sérum du patient capables de réagir avec le complexe substance à forte affinité pour la substance héparinique-substance héparinique (SFA-SH), puis,
2) révéler le complexe produit (Ag-SH)-anti(Ag-SH) résultant après au moins un lavage pour éliminer toute substance pouvant interférer avec le système de détection.

3. 3- Procédé selon la revendication 1,
**caractérisé en ce que** la au moins une substance à forte affinité pour la substance héparinique est choisie parmi le sulfate de protamine, la poly-L-lysine, la poly-arginine, le polybrène ou la streptavidine, la au moins une substance héparinique étant dans ce dernier cas biotinylée.

4. Procédé selon l'une des revendications 1 à 3,
**caractérisé en ce que** la révélation du complexe (Ag-SH)-anti(Ag-SH) est réalisée par des tests EIA (tests enzymo-immunologiques) tels qu'un test ELISA.

5. Procédé selon l'une des revendications 1 à 3
**caractérisé en ce que** la révélation du complexe (Ag-SH)-anti(Ag-SH) est réalisée par des tests RIA (tests radio-immunologiques). 3

6. Procédé selon l'une des revendications 1 à 3,
**caractérisé en ce que** la révélation du complexe (Ag-SH)-anti(Ag-SH) est réalisée par des tests d'immunofluorescence, par néphélométrie, par turbidimétrie, par réfractométrie, par diffraction de rayons lumineux par la surface réactionnelle, par des méthodes de modification de la conductivité électrique ou du champ magnétique, ou toute autre technique de révélation appropriée.

7. **Nécessaire de détection des anticorps héparine-dépendants** et de diagnostic des pathologies immunes ou auto-immunes potentialisées par une substance héparinique choisies parmi la thrombopénie produite par la présence de polysulfate de pentosane ou la thrombopénie induite par l'héparine (TIH de type II) en tant que médicament inducteur, **caractérisé en ce qu'**il comprend :
- au moins une substance à forte affinité pour une substance héparinique (SFA) immobilisée sur un support solide, constituée par toute molécule ou complexe ayant une forte affinité pour une substance héparinique,
- au moins une substance héparinique (SH), choisie parmi l'héparine, les dérivés hépariniques, le polysulfate de pentosane, tout polysaccharide sulfaté chargé négativement ou des polymères de polystyrène sulfatés comme les polymères linéaires non-glycosaminoglycanes chargés négativement n'étant pas des hydrates de carbone, tels que le polyvinylsulfate, le polyvinylsulfonate, le polystyrène sulfonate, le polyanétholsulfonate, des polyvinyl phosphate et polyvinylphosphonate, le poly-D glutamate, ou leurs formes biotinylées.
- un lysat plaquettaire consistant en un concentré de plaquettes normales ou lysées, et/ou un lysat leucocytaire, contenant des molécules affines pour la substance héparinique (SH) potentiellement antigéniques et appelées substances antigéniques potentielles (Ag), sans purification préalable desdites substances antigéniques potentielles et capables de réagir avec la substance héparinique, formant ainsi un complexe (Ag-SH), le complexe substance à forte affinité pour une substance héparinique-substance héparinique (SFA-SH) formé, étant destiné à réagir avec la au moins une substance antigénique non purifiée en quantité suffisante pour être capable de réagir avec une substance héparinique formant ainsi le complexe (Ag-SH) et un sérum ou un plasma contenant potentiellement un matériau anticorps anti(Ag-SH), généré dans l'organisme après administration d'une substance héparinique, en cas de thrombopénie induite par l'héparine, et qui reconnaît Ag-SH, selon la réaction
(Ag-SH) + anti(Ag-SH) → (Ag-SH)-anti(Ag-SH)
et/ou au moins un complexe immun constitué d'un complexe Ag-SH-anticorps anti(Ag-SH) présent dans le plasma ou sérum du patient capables de réagir avec le complexe substance à forte affinité pour la substance héparinique-substance héparinique (SFA-SH).

8. Nécessaire selon la revendication 7
**caractérisé en ce que** ladite au moins une substance à forte affinité pour la substance héparinique est choisie parmi le sulfate de protamine, la poly-L-lysine, la poly-arginine, le polybrène ou la streptavidine, la au moins une substance héparinique étant alors biotinylée.

9. Nécessaire selon l'une des revendications 7 et 8,
**caractérisé en ce que** la au moins une substance héparinique peut être choisie parmi :
- l'héparine non fractionnée (HNF) de poids moléculaire moyen de 6000-30000 daltons et de pouvoir rotatoire [α]_{D}²⁰ d'environ + 55° ;
- l'héparine de bas poids moléculaire ;
- des composés qui dérivent de l'héparine comme les héparinates métalliques (Ca²⁺, Li⁺, Na⁺, Mg²⁺, etc...) et des fragments de l'héparine ;
- des analogues de l'héparine comme les héparinoïdes tels que l'héparamine et ses sels, les chondroitines et leurs sels ;
- les substances contenant l'héparine, ses dérivés et analogues comme les complexes de l'héparine et de ses dérivés ou analogues ;
- leurs mélanges
- l'une quelconque des molécules ci-dessus sous forme biotinylée.

10. Nécessaire selon l'une des revendications 7 à 9,
**caractérisé en ce que** la au moins une substance à forte affinité pour la substance héparinique est fixée sur un support choisi parmi notamment les lames, les micro-plaques, les latex fluorescents, les latex magnétiques, les membranes d'immuno-filtration ou les membranes d'immuno-migration, les biochips, les billes, les ailettes, les tubes, les liposomes, les vésicules lipidiques, les micro-particules biologiques ou obtenues à partir de polymères, ou les émulsions ou tout autre support adapté et ladite fixation étant réalisée telle que par adsorption, par liaisons covalentes.

11. Nécessaire selon l'une des revendications 7 à 10,
**caractérisé en ce qu'**il comprend un lysat plaquettaire consistant en un concentré de plaquettes normales, lysées ou un lysat leucocytaire contenant en excès, la au moins une substance antigénique potentielle capable de réagir avec la au moins une substance héparinique pour former le complexe (Ag-SH).

12. Nécessaire selon l'une des revendications 7 à 11,
**caractérisé en ce qu'**il comporte également le matériel approprié et nécessaire pour révéler le complexe produit.

13. Nécessaire selon la revendication 12,
**caractérisé en ce que** la révélation du complexe est réalisée par des tests EIA (tests enzymo-immunologiques) tels qu'un test ELISA.

14. Nécessaire selon la revendication 12
**caractérisé en ce que** la révélation du complexe est réalisée par toute technique permettant d'identifier et/ou de quantifier une réaction antigène-anticorps ou un complexe immun.

## Claims

1. Method for detecting heparin-dependent antibodies and diagnosing immune or autoimmune diseases potentiated by a heparin substance chosen from pentosan polysulphate-induced thrombocytopenia or heparin-induced thrombocytopenia (HIT type II) as an inductive drug, said method being **characterised in that** it comprises steps consisting of:
1) reacting:
- at least one substance with a high affinity for a heparin substance (HAS) immobilised on a solid substrate, consisting of any molecule or complex having a high affinity for a heparin substance,
- with at least one heparin substance (HS) chosen from heparin, heparin derivatives, negatively charged non-glycosaminoglycan linear non-carbohydrate polymers chosen from polyvinyl sulphate, polyvinyl sulphonate, polystyrene sulphonate, polyanethol sulphonate, polyvinyl phosphate and polyvinyl phosphonate, poly-D-glutamate, to form a high-affinity substance for the heparin substance-heparin substance complex (HAS-HS), the heparin substance being in a slight or large excess, to be able to fix other substances with an affinity for a heparin substance,
- and after at least one washing to remove the non-fixed at least one heparin substance,
- with a platelet lysate consisting of a normal or lysed platelet concentrate and/or a leukocyte concentrate, containing potentially antigenic molecules with an affinity for the heparin substance (HS), referred to as potential antigenic substances (Ag), without prior purification of said potential antigenic substances and capable of reacting with the heparin substance, thus forming a complex (Ag-HS),
- and, finally, with the plasma or serum of a patient under test (diluted, preferably to a dilution in the region of 1:100) potentially containing an anti-(Ag-HS) antibody generated in the body after administering a heparin substance, which recognises (Ag-HS), according to the reaction:
(Ag-HS) + anti(Ag-HS) --> (Ag-HS)-anti(Ag-HS)
and/or at least one immune complex consisting of an Ag-HS-anti(Ag-HS) antibody complex found in the patient's plasma or serum capable of reacting with the high-affinity substance for the heparin substance-heparin substance complex (HAS-HS), and
2) detecting the resulting complex produced (Ag-HS)-anti(Ag-HS) after at least one washing to remove any substance liable to interfere with the detection system.

2. Method for detecting heparin-dependent antibodies and diagnosing immune or autoimmune diseases potentiated by a heparin substance chosen from pentosan polysulphate-induced thrombocytopenia or heparin-induced thrombocytopenia (HIT type II) as an inductive drug, said method being **characterised in that** it consists in the following steps:
1) reacting:
- at least one substance with a high affinity for a heparin substance (HAS) immobilised on a solid substrate and chosen from protamine sulphate, poly-L-lysine, poly-arginine, polybrene or streptavidin, the at least one heparin substance being in the latter case biotinylated,
- with at least one heparin substance (HS) chosen from heparin, heparin derivatives, negatively charged non-glycosaminoglycan linear non-carbohydrate polymers chosen from polyvinyl sulphate, polyvinyl sulphonate, polystyrene sulphonate, polyanethol sulphonate, polyvinyl phosphate and polyvinyl phosphonate, poly-D-glutamate, to form a high-affinity substance for the heparin substance-heparin substance complex (HAS-HS), the heparin substance being in a slight or large excess, to be able to fix other substances with an affinity for a heparin substance,
- and after at least one washing to remove the non-fixed at least one heparin substance,
- finally with the plasma or serum of a patient under test (diluted, preferably to a dilution in the region of 1:100) potentially containing:
an anti-(Ag-HS) antibody generated in the body after administering a heparin substance, which recognises (Ag-HS), according to the reaction:
(Ag-HS) + anti(Ag-HS) --> (Ag-HS)-anti(Ag-HS)
and/or at least one immune complex consisting of an Ag-HS-anti(Ag-HS) antibody complex found in the patient's plasma or serum capable of reacting with the high-affinity substance for the heparin substance-heparin substance complex (HAS-HS), and
2) detecting the resulting complex produced (Ag-HS)-anti(Ag-HS) after at least one washing to remove any substance liable to interfere with the detection system.

3. Method according to claim 1, **characterised in that** the at least one high-affinity substance for the heparin substance is chosen from protamine sulphate, poly-L-lysine, poly-arginine, polybrene or streptavidin, the at least one heparin substance being in the latter case biotinylated.

4. Method according to any of claims 1 to 3, **characterised in that** the complex (Ag-HS)-anti(Ag-HS) is detected by means of EIA (enzyme immunoassay) tests, such as an ELISA test.

5. Method according to any of claims 1 to 3, **characterised in that** the complex (Ag-HS)-anti(Ag-HS) is detected by means of RIA (radioimmunoassay) tests.

6. Method according to any of claims 1 to 3, **characterised in that** the complex (Ag-HS)-anti(Ag-HS) is detected by means of immunofluorescence tests, by means of nephelometry, turbidimetry, refractometry, light ray diffraction by the reaction surface, electrical conductivity or magnetic field modification methods, or any other suitable detection technique.

7. Kit for detecting heparin-dependent antibodies and diagnosing immune or autoimmune diseases potentiated by a heparin substance chosen from pentosan polysulphate-induced thrombocytopenia or heparin-induced thrombocytopenia (HIT type II) as an inductive drug, **characterised in that** it comprises:
- at least one substance with a high affinity for a heparin substance (HAS) immobilised on a solid substrate, consisting of any molecule or complex having a high affinity for a heparin substance,
- at least one heparin substance (HS) chosen from heparin, heparin derivatives, any negatively charged sulphated polysaccharide or sulphated polystyrene polymers such as negatively charged non-glycosaminoglycan linear non-carbohydrate polymers, such as polyvinyl sulphate, polyvinyl sulphonate, polystyrene sulphonate, polyanethol sulphonate, polyvinyl phosphate and polyvinyl phosphonate, poly-D-glutamate, or the biotinylated forms thereof,
- a platelet lysate consisting of a normal or lysed platelet concentrate and/or a leukocyte concentrate, containing potentially antigenic molecules with an affinity for the heparin substance (HS), referred to as potential antigenic substances (Ag), without prior purification of said potential antigenic substances and capable of reacting with heparin substance, thus forming a complex (Ag-HS),
the high-affinity substance for a heparin substance-heparin substance complex (HAS-HS) formed, being intended to react with the at least one non-purified antigenic substance in a sufficient amount to be capable of reacting with a heparin substance thus forming the (Ag-HS) complex and a serum or a plasma potentially containing an anti(Ag-HS) antibody material generated in the body after administering a heparin substance, in the case of heparin-induced thrombocytopenia, which recognises Ag-HS, according to the reaction:
(Ag-HS) + anti(Ag-HS) → (Ag-HS)-anti(Ag-HS)
and/or at least one immune complex consisting of an Ag-HS-anti(Ag-HS) antibody complex found in the patient's plasma or serum capable of reacting with the high-affinity substance for the heparin substance-heparin substance complex (HAS-HS).

8. Kit according to claim 7, **characterised in that** said at least one high-affinity substance for the heparin substance is chosen from protamine sulphate, poly-L-lysine, poly-arginine, polybrene or streptavidin, the at least one heparin substance being biotinylated in this case.

9. Kit according to any of claims 7 and 8, **characterised in that** the at least one heparin substance can be chosen from:
- non-fractionated heparin (NFH) having a mean molecular weight of 6000-30,000 Daltons and a rotatory power [α]_{D}²⁰ of approximately +55°;
- low molecular weight heparin;
- compounds derived from heparin such as metal heparinates (Ca²⁺, Li⁺, Na⁺, Mg²⁺, etc.) or heparin fragments;
- heparin analogues such as heparinoids such as heparamine and the salts thereof, chondroitins and the salts thereof;
- substances containing heparin, the derivatives and analogues thereof such as complexes of heparin and the derivatives or analogues thereof;
- mixtures thereof;
- any of the above molecules in biotinylated form.

10. Kit according to any of claims 7 to 9, **characterised in that** the at least one high-affinity substance for the heparin substance is fixed on the substrate chosen from, in particular, slides, microplates, fluorescent latexes, magnetic latexes, immunofiltration membranes or immuno-migration membranes, biochips, beads, blades, tubes, liposomes, lipid vesicles, biological or polymer-derived micro-particles, or emulsions or any other suitable substrate and said fixing being obtained by means of adsorption, covalent bonds, for example.

11. Kit according to any of claims 7 to 10, **characterised in that** it comprises a platelet lysate consisting of a normal, lysed platelet concentrate or a leukocyte lysate containing, in excess, the at least one potential antigenic substance capable of reacting with the at least one heparin substance to form the complex (Ag-HS).

12. Kit according to any of claims 7 to 11, **characterised in that** it also includes the suitable materials required to detect the complex produced.

13. Kit according to claim 12, **characterised in that** the complex is detected by means of EIA (enzyme immunoassay) tests, such as an ELISA test.

14. Kit according to claim 12, **characterised in that** the complex is detected by means of any technique for identifying and/or quantifying an antigen-antibody reaction or an immune complex.

## Patentansprüche

1. Verfahren für den Nachweis heparinabhängiger Antikörper und die Diagnose von Immun- bzw. Autoimmunerkrankungen, verstärkt durch eine Heparinsubstanz, ausgewählt aus der Thrombopenie, erzeugt durch die Anwesenheit von Pentosanpolysulfat, oder der Heparin-induzierten Thrombopenie (TIH vom Typ II) als Induktorarzneimittel, wobei das Verfahren **dadurch gekennzeichnet ist, dass** es die folgenden Schritte umfasst:
1) Reagieren:
- mindestens einer Substanz mit einer starken Affinität für eine Heparinsubstanz (SFA), immobilisiert auf einem festen Träger, bestehend aus jedem Molekül oder Komplex mit einer starken Affinität für eine Heparinsubstanz,
- mit mindestens einer Heparinsubstanz (SH), ausgewählt aus Heparin, den Heparinderivaten, den negativ geladenen linearen Nicht-Glycosaminoglycan-Polymeren, die kein Kohlenhydrat sind, ausgewählt aus dem Polyvinylsulfat, dem Polyvinylsulfonat, dem Polystyrensulfonat, dem Polyanetholsulfonat, dem Polyvinylphosphat und Polyvinylphosphonat, dem Poly-D Glutamat, um eine komplexe Substanz mit starker Affinität für die Heparinsubstanz-Heparinsubstanz (SFA-SH) zu bilden, wobei die Heparinsubstanz in geringfügigem oder großem Überschuss vorhanden ist, um andere Substanzen, die für eine Heparinsubstanz affin sind, anlagern zu können,
- dann, nach mindestens einer Waschung, um die mindestens eine, nicht angelagerte Heparinsubstanz zu eliminieren,
- mit einem Plättchenlysat, bestehend aus einem Konzentrat von normalen oder lysierten Plättchen und/oder einem Leukozytenlysat, umfassend für die Heparinsubstanz (SH) affine, potenziell antigene und als potenziell antigene Substanzen (Ag) bezeichnete Moleküle, ohne vorherige Reinigung der potenziellen antigenen Substanzen und dazu in der Lage, mit der Heparinsubstanz zu reagieren, wobei auf diese Weise ein Komplex (Ag-SH) gebildet wird,
- und schließlich mit dem Plasma oder dem Serum eines zu testenden Patienten (vorzugsweise zu einer Verdünnung im Bereich von 1:100 verdünnt), umfassend potenziell:
ein Antikörper-Anti(Ag-SH)-Material, erzeugt im Organismus nach der Verabreichung einer Heparinsubstanz, und das (Ag-SH) erkennt, gemäß der folgenden Reaktion:
(Ag-SH) + anti(Ag-SH) --> (Ag-SH)-anti(Ag-SH)
und/oder mindestens einen Immunkomplex, bestehend aus einem Ag-SH-Antikörper-Anti(Ag-SH)-Komplex, der im Plasma oder im Serum des Patienten vorhanden ist, dazu in der Lage, mit dem Komplex Substanz mit starker Affinität für die Heparinsubstanz-Heparinsubstanz (SFA-SH) zu reagieren, dann
2) Erfassen des erzeugten Komplexes (Ag-SH)-Anti(Ag-SH), der sich nach mindestens einer Waschung ergibt, um jede Substanz zu entfernen, die das Nachweissystem beeinflussen kann.

2. Verfahren für den Nachweis heparinabhängiger Antikörper und die Diagnose von Immun- bzw. Autoimmunerkrankungen, verstärkt durch eine Heparinsubstanz, ausgewählt aus der Thrombopenie, erzeugt durch die Anwesenheit von Pentosanpolysulfat oder der Heparin-induzierten Thrombopenie (TIH vom Typ II) als Induktorarzneimittel, wobei das Verfahren **dadurch gekennzeichnet ist, dass** es in die folgenden Schritte besteht:
1) Reagieren:
- mindestens einer Substanz mit einer starken Affinität für eine Heparinsubstanz (SFA), immobilisiert auf einem festen Träger und ausgewählt aus dem Protaminsulfat, dem Poly-L-Lysin, dem Poly-Arginin, dem Polybren oder dem Streptavidin, wobei die mindestens eine Heparinsubstanz in diesem letzteren Fall biotinyliert ist,
- mit mindestens einer Heparinsubstanz (SH), ausgewählt aus Heparin, den Heparinderivaten, den negativ geladenen linearen Nicht-Glycosaminoglycan-Polymeren, die kein Kohlenhydrat sind, ausgewählt aus dem Polyvinylsulfat, dem Polyvinylsulfonat, dem Polystyrensulfonat, dem Polyanetholsulfonat, dem Polyvinylphosphat und Polyvinylphosphonat, dem Poly-D Glutamat, um eine komplexe Substanz mit starker Affinität für die Heparinsubstanz-Heparinsubstanz (SFA-SH) zu bilden, wobei die Heparinsubstanz in geringfügigem oder großem Überschuss vorhanden ist, um andere Substanzen, die für eine Heparinsubstanz affin sind, anlagern zu können,
- dann, nach mindestens einer Waschung, um die mindestens eine nicht angelagerte Heparinsubstanz zu eliminieren,
- schließlich mit dem Plasma oder dem Serum eines zu testenden Patienten (vorzugsweise zu einer Verdünnung im Bereich von 1:100 verdünnt), umfassend potenziell:
- ein Antikörper-Anti(Ag-SH)-Material, erzeugt im Organismus nach der Verabreichung einer Heparinsubstanz, und das (Ag-SH) erkennt, gemäß der folgenden Reaktion:
(Ag-SH) + anti(Ag-SH) --> (Ag-SH)-anti(Ag-SH)
und/oder mindestens einem Immunkomplex, bestehend aus einem Ag-SH-Antikörper-Anti(Ag-SH)-Komplex, der im Plasma oder im Serum des Patienten vorhanden ist, dazu in der Lage, mit dem Komplex Substanz mit starker Affinität für die Heparinsubstanz-Heparinsubstanz (SFA-SH) zu reagieren, dann
2) Erfassen des erzeugten Komplexes (Ag-SH)-Anti(Ag-SH), der sich nach mindestens einer Waschung ergibt, um jede Substanz zu entfernen, die das Nachweissystem beeinflussen kann,

3. Verfahren nach Anspruch 1,
**dadurch gekennzeichnet, dass** die mindestens eine Substanz mit starker Affinität für die Heparinsubstanz ausgewählt ist unter dem Protaminsulfat, dem Poly-L-Lysin, dem Poly-Arginin, dem Polybren oder dem Streptavidin, wobei die mindestens eine Heparinsubstanz in diesem letzteren Fall biotinyliert ist.

4. Verfahren nach einem der Ansprüche 1 bis 3,
**dadurch gekennzeichnet, dass** die Erfassung des Komplexes (Ag-SH)-anti(Ag-SH) durch EIA-Tests (enzymimmunologische Tests) wie z.B. einem ELISA-Test erfolgt.

5. Verfahren nach einem der Ansprüche 1 bis 3,
**dadurch gekennzeichnet, dass** die Erfassung des Komplexes (Ag-SH)-anti(Ag-SH) durch RIA-Tests (radioimmunologische Tests) erfolgt.

6. Verfahren nach einem der Ansprüche 1 bis 3,
**dadurch gekennzeichnet, dass** die Erfassung des Komplexes (Ag-SH)-anti(Ag-SH) durch Immunofluoreszenz-Tests, Nephelometrie, Turbidimetrie, Refraktometrie, Diffraktion von Lichtstrahlen durch die Reaktionsoberfläche, Verfahren der Veränderung der elektrischen Leitfähigkeit oder des Magnetfeldes, oder jede andere geeignete Nachweistechnik erfolgt.

7. Kit für den Nachweis heparinabhängiger Antikörper und die Diagnose von Immun- bzw. Autoimmunerkrankungen, verstärkt durch eine Heparinsubstanz, ausgewählt aus der Thrombopenie, erzeugt durch die Anwesenheit von Pentosanpolysulfat, oder der Heparin-induzierten Thrombopenie (TIH vom Typ II) als Induktorarzneimittel, **dadurch gekennzeichnet, dass** es Folgendes umfasst:
- mindestens eine Substanz mit einer starken Affinität für eine Heparinsubstanz (SFA), immobilisiert auf einen festen Träger, bestehend aus jedem Molekül oder Komplex mit einer starken Affinität für eine Heparinsubstanz,
- mindestens eine Substanz mit starker Heparinaffinität (SH), ausgewählt aus Heparin, den Heparinderivaten, dem Pentosanpolysulfat, jedem negativ geladenen Polysaccharidsulfat oder Polystyrensulfatpolymeren wie z.B. negativ geladenen linearen Nicht-Glycosaminoglycan-Polymeren, die keine Kohlenhydrate sind, wie z.B. Polyvinylsulfat, Polyvinylsulfonat, Polystyrensulfonat, Polyanetholsulfonat, Polyvinylphosphat und Polyvinylphosphonat, Poly-D Glutamat oder ihre biotinylierten Formen.
- ein Plättchenlysat, bestehend aus einem Konzentrat von normalen oder lysierten Plättchen und/oder einem Leukozytenlysat, umfassend für die Heparinsubstanz (SH) affine, potenziell antigene und als potenziell antigene Substanzen (Ag) bezeichnete Moleküle, ohne vorherige Reinigung der potenziellen antigenen Substanzen und dazu in der Lage, mit der Heparinsubstanz zu reagieren, wobei auf diese Weise ein Komplex (Ag-SH) gebildet wird, wobei der gebildete Komplex Substanz mit starker Affinität für die Heparinsubstanz-Heparinsubstanz (SFA-SH) dazu vorgesehen ist, mit der mindestens einen nicht gereinigten antigenen Substanz in ausreichender Menge zu reagieren, um dazu in der Lage zu sein, mit einer Heprinsubstanz zu reagieren, wobei so der Komplex (Ag-SH) gebildet wird, und einem Serum oder Plasma, enthaltend potenziell ein Antikörper-Anti(Ag-SH)-Material, das im Organismus nach der Verabreichung einer Heparinsubstanz erzeugt wird, im Fall von Heparin-induzierter Thrombopenie, und das Ag-SH erkennt, gemäß der folgenden Reaktion:
(Ag-SH) + anti(Ag-SH) → (Ag-SH)-anti(Ag-SH)
und/oder mindestens ein Immunkomplex, bestehend aus einem Komplex Ag-SH-Antikörper-Anti(Ag-SH), der im Plasma oder im Serum des Patienten vorhanden ist, dazu in der Lage, mit dem Komplex Substanz mit starker Affinität für die Heparinsubstanz-Heparinsubstanz (SFA-SH) zu reagieren.

8. Kit nach Anspruch 7,
**dadurch gekennzeichnet, dass** die mindestens eine Substanz mit starker Affinität für die Heparinsubstanz ausgewählt ist unter dem Protaminsulfat, dem Poly-L-Lysin, dem Poly-Arginin, dem Polybren oder dem Streptavidin, wobei die mindestens eine Heparinsubstanz dann biotinyliert ist.

9. Kit nach einem der Ansprüche 7 und 8,
**dadurch gekennzeichnet, dass** die mindestens eine Heparinsubstanz unter Folgendem ausgewählt werden kann:
- nicht fraktioniertem Heparin (HNF) mit einem mittleren Molekulargewicht von 6 000 - 30 000 Dalton und einem Drehvermögen [α]_{D}²⁰ von ungefähr + 55°;
- Heparin mit niedrigem Molekulargewicht;
- Zusammensetzungen, die sich vom Heparin ableiten, wie z.B. metallische Heparinate (Ca²⁺, Li⁺, Na⁺, Mg²⁺ usw.) und Fragmente von Heparin.
- Analoge von Heparin wie z.B. Heparinoide wie Heparamin und seine Salze, Chondroitine und ihre Salze;
- die Substanzen, die Heparin enthalten, seine Derivate und Analoge wie die Komplexe des Heparins und seiner Derivate oder Analoge;
- ihre Mischungen
- eines der oben angegebenen Moleküle in biotinylierter Form.

10. Kit nach einem der Ansprüche 7 bis 9,
**dadurch gekennzeichnet, dass** die mindestens eine Substanz mit starker Affinität für die Heparinsubstanz auf einem Träger angelagert ist, ausgewählt insbesondere aus Klingen, Mikroplatten, fluoreszentem Latex, magnetischem Latex, Immunofiltrationsmembranen oder Immunomigrationsmembranen, Biochips, Kugeln, Rippen, Schläuchen, Liposomen, Fettvesikeln, biologischen Mikropartikeln oder erhalten aus Polymeren, oder Emulsionen oder jedem anderen angepassten Träger, und wobei die Anlagerung z.B. durch Adsorption, durch covalente Bindungen, durchgeführt ist.

11. Kit nach einem der Ansprüche 7 bis 10,
**dadurch gekennzeichnet, dass** es ein Plättchenlysat umfasst, bestehend aus einem Konzentrat von normalen, lysierten Plättchen oder einem Leukozytenlysat, enthaltend einen Überschuss der mindestens einen potenziellen antigenen Substanz, die dazu in der Lage ist, mit der mindestens einen Heparinsubstanz zu reagieren, um den Komplex (Ag-SH) zu bilden.

12. Kit nach einem der Ansprüche 7 bis 11,
**dadurch gekennzeichnet, dass** es auch das geeignete und für die Erfassung des produzierten Komplexes erforderliche Material umfasst.

13. Kit nach Anspruch 12,
**dadurch gekennzeichnet, dass** die Erfassung des Komplexes durch EIA-Tests (enzymimmunologische Tests) wie z.B. einem ELISA-Test erfolgt.

14. Kit nach Anspruch 12,
**dadurch gekennzeichnet, dass** die Erfassung des Komplexes durch jede Technik erfolgt, die ermöglicht, eine Antigen-Antikörper-Reaktion oder einen Immunkomplex zu identifizieren und/oder zu quantifizieren.
